Europäisches Patentamt

European Patent Office

Office européen des brevets

(11) Veröffentlichungsnummer: **0 436 840 A2**

(12) **EUROPÄISCHE PATENTANMELDUNG**

(21) Anmeldenummer: 90123500.2

(22) Anmeldetag: 07.12.90

(51) Int. Cl.⁵: **A61N 1/16**

(30) Priorität: 06.01.90 DE 9000088 U
30.03.90 DE 4010240

(43) Veröffentlichungstag der Anmeldung:
**17.07.91 Patentblatt 91/29**

(84) Benannte Vertragsstaaten:
**AT BE CH DE DK ES FR GB GR IT LI LU NL SE**

(71) Anmelder: **RAYONEX WELLENTECHNIK GMBH**
**Winterberger Strasse 66**
**W-5940 Lennestadt 1(DE)**

(72) Erfinder: **Schmidt, Paul, Dipl.-Ing.**
**Reiherstrasse 1**
**W-5940 Lennestadt 1 - Saalhausen(DE)**

(74) Vertreter: **Grosse, Dietrich, Dipl.-Ing. et al**
**Patentanwälte**
**HEMMERICH-MÜLLER-GROSSE-POLLMEIER--**
**MEY-VALENTIN Hammerstrasse 2**
**W-5900 Siegen 1(DE)**

(54) **Resonator für hohe Frequenzen.**

(57) Ein Resonator für hohe Frequenzen mit in aus Isolierstoff bestehenden Brücken 6, 1 einander parallel angeordneten Leiterstäben 3, bei dem die Resonanzfrequenz durch gegenseitiges Schränken zweier Isolierbrücken und damit der Leiterstäbe einstellbar ist, soll derart weitergebildet werden, daß der Resonator eine Mehrzahl von Resonanzfrequenzen, und zwar mindestens zwei Resonanzfrequenzen, aufweist, ohne daß ein wesentlich gesteigerter Aufwand zu treiben ist.

Hierfür wird der Resonator für jede zusätzliche Resonanzfrequenz mit einer weiteren, Leiterstäbe 3 aufweisenden Isolierbrücke 1 bestückt, und diese zusätzliche Resonanzfrequenz ist durch Schränken der weiteren Isolierbrücke 1 gegen die unter dieser angeordnete einstellbar.

Fig. 5

EP 0 436 840 A2

# RESONATOR FÜR HOHE FREQUENZEN

Die Erfindung betrifft einen Resonator für hohe Frequenzen mit in aus Isolierstoff bestehenden Brücken einander parallel angeordneten Leiterstäben.

Derartige Resonatoren sind bereits benutzt worden, um Krankheiten zu beheben oder wenigstens zu mildern bzw. das Wohlbefinden zu steigern.

Die Schulmedizin benutzt für Heilverfahren verbreitet Strahlungen, die teils elektromagnetischer Natur sind wie Wärmestrahlen bis Gammastrahlen, die mechanischer Natur sind, wie Schall und Ultraschall, oder die in Verbindung mit dem Atom-zerfall auftreten, wie Alpha-, Beta- und Gammastrahlen. Mittels der gattungsgemäßen Resonatoren lassen sich darüber hinaus Schwingungen im Bereich der Bioenergien nutzen, die jedoch nach dem heutigen Stand der Physik sich objektiv ebensowenig erfassen und anmessen lassen wie bspw. die vermittels Wünschelruten erfaßbaren sogenannten Erdstrahlen von Erz- oder Wasseradern. Die gewünschte Resonanzfrequenz solcher Resonatoren jedoch läßt sich mittels Vergleich mit Generatoren elektromagnetischer Wellen bewirken, wobei Pendel oder dergleichen als die Resonanz anzeigende Indikatoren wirken.

Die Erfindung geht von der Aufgabe aus, ein Gerät zu schaffen, mit dessen Hilfe sich jene Schwingungen willkürlich und mit ausgesuchten Frequenzen zur Wirkung bringen lassen, um Lebensvorgänge positiv zu beeinflussen und insbesondere Krankheiten zu heilen, mindestens aber in ihren Auswirkungen abzuschwächen und zu lindern, wobei davon ausgegangen wird, daß bei bestimmten Erkrankungen eine Abstellung der Einwirkung auf eine Frequenz nicht ausreichend ist, sondern die Schwingungen mit zwei, gegebenenfalls auch weiteren Frequenzen wirksam sein sollten. Die Aufgabe im engeren Sinne zielt daher darauf ab, einen Resonator zu schaffen, der leicht und einfach herstellbar ist, und dessen Resonanzverhalten zwei bzw. weitere Resonanzbereiche bzw. Resonanzfrequenzen aufweist.

Gelöst wird diese Aufgabe mit den kennzeichnenden Merkmalen des Schutzanspruches 1. Die in parallelen Ebenen übereinander angeordneten Elemente, nämlich eine Leiterplatte und drei Isolierbrücken, die jeweils einander parallel angeordnete und sich rechtwinklig zur Längsrichtung der Brücke erstreckende Leiterstäbe enthalten, gestatten eine Ein- und Auskopplung über die Leiterplatte, und die Winkeleinstellungen zwischen den Leiterstäben der einzelnen Brücken gestatten eine Einstellung der gewünschten Resonanzfrequenzen, wobei einerseits der für den Aufbau des Resonators zu treibende Aufwand erträglich ist und andererseits sich mehrere, bspw. zwei, Resonanzfrequenzen beliebig einstellen lassen, und schließlich durch das relativ kleine und leichte, von einem Gehäuse umschlossene Gerät sich eine leichte und einfache Handhabung desselben ergibt.

Zweckmäßige Weiterbildungen sind in den Unteransprüchen gekennzeichnet und erleichtern die Einstellbarkeit und sichern eine erfolgte Einstellung, sie gestatten die Berücksichtigung weiterer, bspw. bis zu zehn, Resonanzfrequenzen bei geringem zusätzlichem Aufwande, und sie erleichtern die Einwirkung auf einen krankheitsbehafteten Körper durch Wahl der jeweils optimalen Elektroden.

Im einzelnen sind die Merkmale des Resonators anhand der folgenden Beschreibung eines Ausführungsbeispieles in Verbindung mit dieses darstellenden Zeichnungen erläutert. Es zeigen hierbei

Figur 1     die Aufsicht auf eine mit Leiterstäben bestückte Isolierbrücke,

Figur 2     eine Seitenansicht der Isolierbrücke nach Fig. 1,

Figur 3     die Aufsicht auf eine Leiterplatte,

Figur 4     die Seitenansicht eines montierten, für zwei Frequenzen vorgesehenen Resonators,

Figur 5     die Aufsicht auf den Resonator nach Fig. 4,

Figur 6     die Aufsicht auf das Gehäuse des Resonators in Verbindung mit an ihn anschließbare Elektroden, und

Figur 7     die Aufsicht auf ein geöffnetes, die untere Isolierbrücke zentrierendes Gehäuse.

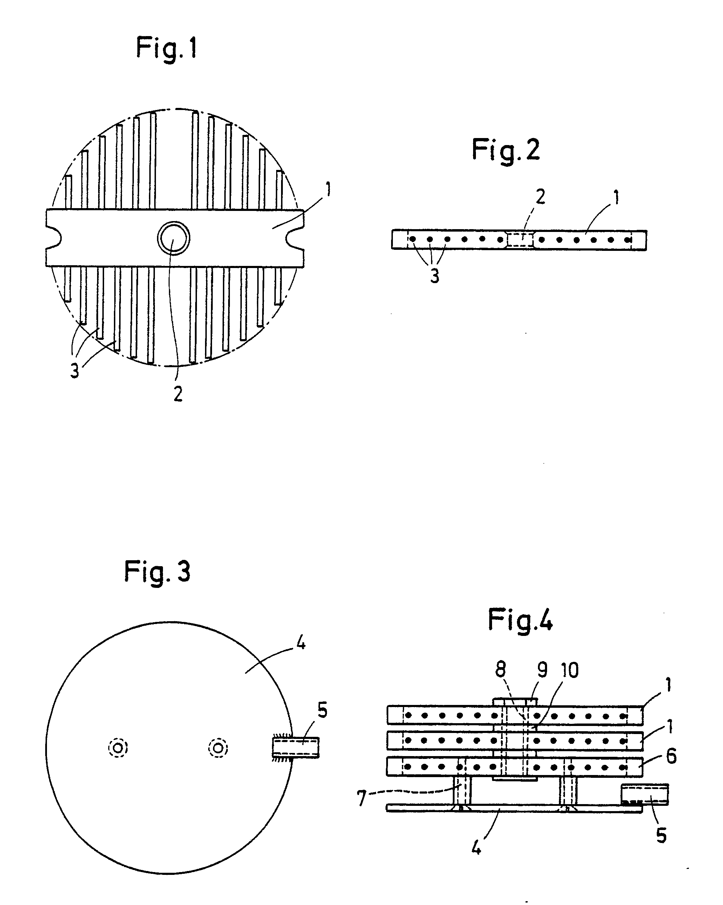

In den Figuren 1 und 2 sind die Aufsicht und die Ansicht einer Isolierbrücke 1 gezeigt, die aus einem isolierenden Kunststoff, wie Acrylglas, Polystyrol, Trolitul oder dergleichen besteht. Mittig der Isolierbrücke 1 der Fig. 1 ist eine Vertikalbohrung 2 vorgesehen, und in beidseitig dieser Vertikalbohrung 2 äquidistant vorgesehene Querbohrungen sind Leiterstäbe 3 eingefügt, deren Länge so bemessen ist, daß sie sich bis zu einem gemeinsamen Umkreis erstrecken.

In Fig. 3 ist die Aufsicht auf eine kreisförmige Leiterplatte 4 gezeigt, die mit einer Anschlußbuchse 5 versehen ist.

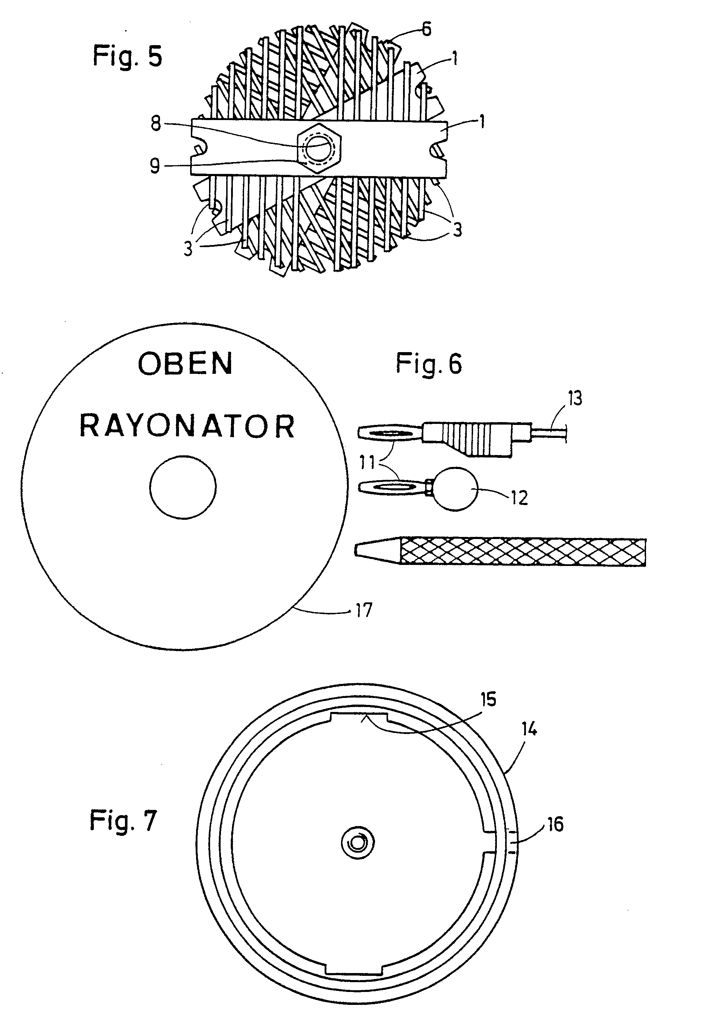

Der Zusammenbau des eigentlichen Resonators ohne das ihn umschließende Gehäuse ist anhand der Fig. 4 veranschaulicht. Eine erste Isolierbrücke 6 ist, zweckmäßig über Distanzstücke, mit der Leiterplatte 4 durch Schrauben 7 verbunden. Die Vertikalbohrung der Isolierbrücke 6 ist von einer Gewindebuchse 8 durchgriffen, welche Distanzringe 10 sowie zwei weitere Isolierbrücken 1 durchgreift. Gegeneinander verspannbar sind die Isolierbrücken mittels einer auf die Gewindebuchse 8 aufgebrachten Mutter 9. Damit ergibt sich insgesamt eine Aufsicht entsprechend der Fig. 5.

Gegen Umwelteinflüsse wird der Resonatoraufbau durch ein Gehäuse geschützt. In Fig. 7 ist ein Gehäuseunterteil 14 dargestellt, das Aussparungen 15, 16 für die Anschlußbuchse 5 sowie die beiden Enden der Isolierbrücke 6 aufweist. Nach Einbringen wird das Gehäuse durch ein glokkenförmiges Gehäuseoberteil 17 nach Fig. 6 abgedeckt.

Die Einstellung der gewünschten Frequenzbereiche erfolgt nach der Montage der Isolierbrücken des Resonators und vor dem endgültigen Verspannen derselben. Bei noch relativ lockerer Mutter 9 läßt sich die untere der Isolierbrücken 1 gegen die Isolierbrücke 6 derart verdrehen, daß die jeweiligen Leiterstäbe einen Winkel miteinander einschließen, welcher die Resonanzfrequenz bestimmt. Anschließend läßt sich die obere der Isolierbrücken 1 gegen die darunterliegende verdrehen, bis der zwischen den Leiterstäben dieser beiden Isolierbrücken eingeschlossene Winkel der gewünschten zweiten Resonanzfrequenz entspricht. Nun läßt sich die Mutter 9 unter gleichzeitigem Halten der Isolierbrücken festspannen, so daß die gegenseitigen Winkellagen der zentral befestigten Isolierbrücken den gewünschten Resonanzfrequenzen entsprechen. Soll der Resonator für weitere Resonanzfrequenzen ausgelegt werden, so werden entsprechende weitere, Leiterstäbe 3 aufweisende Isolierbrücken 1 vorgesehen und gegen die jeweils darunter liegenden eingestellt.

Es ist auch möglich, jeweils zwischen Isolierbrücken Muttern vorzusehen, mit denen die Isolierbrücken nacheinander durch Anspannen fixiert sind, wobei die jeweils darüberliegende Isolierbrükke sich nach Spannen der darunterliegenden noch drehen läßt, bis sie mit der sie übergreifenden Mutter fixiert wird.

Als zweckmäßig hat es sich erwiesen, jeweils fixierte Isolierbrücken und/oder die die Fixierung bewirkenden Muttern durch einen entsprechenden Fixierlack zu sichern. In jedem dieser Fälle läßt sich damit der Resonator auf zwei bzw. weitere Resonanzfrequenzen einstellen, die mit Rücksicht auf die zu heilende bzw. zu lindernde Krankheit gewählt sind, so daß der Benutzer des Resonators nur nach Anweisung den Resonator gegen die zu behandelnden Körperstellen zu führen hat, so daß diese über den Resonator durch Schwingungen beeinflußt werden, ohne daß das "Innenleben" des Resonators zu beachten ist oder Eingriffe in diesen oder auch nur Einstellungen desselben vorzunehmen sind. Im Falle des Einsatzes eines bezüglich seiner Frequenzen auf Rheuma und den zugehörigen Calzium-Stoffwechsel eingestellten Resonators ist es nur erforderlich, daß der Resonator oder an ihn angeschlossene Elektroden in unmittelbare Nähe der sogenannten Rheuma-Depots gebracht wird.

Die Behandlung selbst wird durch die Möglichkeit, unterschiedliche Elektroden anzuschließen, erleichtert. So können bspw. zur lokalen Behandlung einfache Kugelelektroden 12 vermittels eines zugeordneten Steckers 11 an die Anschlußbuchse angeschlossen werden; es ist aber auch möglich, flächenhafte Elektroden als Sessel/Bett-Elektroden über Kabel 13 anzuschließen oder Stabelektroden zu benutzen. Hierbei ergibt sich die Möglichkeit, bspw. bei einer Rheumabehandlung vermittels eines auf die beiden bei Rheuma wirksamen Frequenzen eingestellten Resonators mit einer Behandlungszeit von bspw. täglich 15 bis 30 Sekunden pro Depot, die das Rheuma bewirkenden und den Körper belastenden Giftstoffe mit einer relativ begrenzten Behandlungsdauer zu neutralisieren, zu lösen und schließlich über Blase, Darm, Schleimhäute und gegebenenfalls auch über die Haut auszuscheiden, ein Resultat, das bisher praktisch nicht oder nur unter großem Aufwand unvollkommen erlangt wurde, so daß mittels des Resonators ein Ergebnis erreichbar ist, welches bisher durch oral zu nehmende Medikamente, Spritzen, Bäderkuren und dergleichen in Verbindung mit vorgegebener Gymnastik nicht erreicht wurde.

Der Aufbau des Resonators läßt sich variieren. So kann der zentrale Zusammenhalt durch eine aus Kunststoff bestehende Gewindebuchse oder eine Schraube bewirkt werden, und die Gewindebuchse 8 bzw. die Schraube kann auch die Leiterplatte 4 mit spannen, so daß die Schrauben 7 entbehrlich werden, oder die Leiterbrücken 1, 1, 6 mitsamt der Leiterplatte 4 gegen das Gehäuseunterteil 14 spannen. Das Gehäuseunterteil 14 kann mit dem Gehäuseoberteil 17 durch Klemmsitz, durch formschlüpssiges Einrasten von Vorsprüngen in Nute, mittels in deren Seitenwandungen eingearbeitete Gewinde oder durch Verkleben der einander umfassenden Seitenwandungen verbunden sein. Sie können aber auch durch zentrales Verschrauben miteinander verbunden sein, und zwar mittels in Innengwinde der Gewindebuchse 8 eingreifende Schrauben, mittels einer die Gewindebuchse durchgreifenden Schraube oder durch die Gewindebuchse selbst bzw. eine entsprechende Schraube. Schließlich kann der Resonator für weitere Frequenzen ausgebildet werden. , indem eine entsprechende Anzahl weiterer Leiterbrücken vorgesehen wird.

## Patentansprüche

1. Resonator für hohe Frequenzen mit in aus Isolierstoff bestehenden Brücken einander parallel angeordneten Leiterstäben,

**dadurch gekennzeichnet,**

daß in parallelen Ebenen übereinander eine Leiterplatte (4) und mindestens drei Isolierbrücken (1, 1, 6) angeordnet sind, wobei die Isolierbrücken jeweils einander parallel zur Längsrichtung der Brücke sich rechtwinklig erstreckende Leiterstäbe (3) aufweisen, und daß die Brücken (1, 1, 6) zentrisch miteinander verbunden und in vorgegebenen gegenseitigen Winkelstellungen fixierbar sind.

2. Resonator nach Anspruch 1,

**dadurch gekennzeichnet,**

daß die Anzahl der Isolierbrücken (1, 6) die der gewünschten Sesonanzfrequenzen um Eins übersteigt.

3. Resonator nach Anspruch 1 oder 2,

**dadurch gekennzeichnet,**

daß die über der Leiterplatte (4) angeordnete Isolierbrücke (6) mit dieser vermittels zweier außerhalb der Mitte von deren Längsachse angeordneter Schrauben (7) verbunden ist, und daß die über dieser angeordneten Isolierbrücken(1) mit ihr mittels einer mittig der Längsachse durchgehenden Schraube verbunden und verspannt sind.

4. Resonator nach Anspruch 3,

**gekennzeichnet durch**

eine als Schraube vorgesehene Gewindebuchse (8).

5. Resonator nach einem der Ansprüche 1 bis 4,

**dadurch gekennzeichnet,**

daß die Leiterplatte (4) mit einer Anschlußbuchse (5) für den Anschluß von Elektroden ausgestattet ist.

7. Resonator nach einem der Ansprüche 1 bis 6,

**gekennzeichnet durch**

gestreckte, mindestens außerhalb der Vertikalbohrung äquidistant vorgesehene Leiterstäbe (3).

7. Resonator nach einem der Ansprüche 1 bis 6,

**dadurch gekennzeichnet,**

daß die Längen der Leiterstäbe (3) jeweils von der Mitte der Isolierbrücke (1, 6) nach außen hin abnehmen.

8. Resonator nach einem der Ansprüche 1 bis 7,

**dadurch gekennzeichnet,**

daß die Leiterplatte (4) und die durch die Enden der Leiterstäbe (3) der Isolierbrücken (1, 6) beschriebenen Konturen jeweils kreisförmig ausgebildet sind.

9. Resonator nach einem der Ansprüche 1 bis 8,

**dadurch gekennzeichnet,**

daß er von einem dosenförmigen Isoliergehäuse umschlossen ist.

10. Resonator nach einem der Ansprüche 1 bis 9,

**dadurch gekennzeichnet,**

daß die Winkelstellung einer der Isolierbrücken (6) durch Ausnehmungen (15) und/oder Vorsprünge des Isoliergehäuses bestimmt ist.

11. Resonator nach einem der Ansprüche 1 bis 10,

**gekennzeichnet durch**

eine mit einem Stecker (11) verbundene Kugelelektrode (12).

12. Resonator nach einem der Ansprüche 1 bis 11,

**gekennzeichnet durch**

eine mit einem Stecker (11) über ein Kabel (13) verbundene Flächenelektrode.

# Fig.1

# Fig.2

# Fig. 3

# Fig.4

**Fig. 5**

8

9

5

1

1

3

3

3

**OBEN**

**RAYONATOR**

**Fig. 6**

13

11

12

17

**Fig. 7**

15

14

16